(19) Europäisches Patentamt
European Patent Office
Office européen des brevets

(11) Publication number : **0 485 217 A2**

(12) **EUROPEAN PATENT APPLICATION**

(21) Application number : **91310320.6**

(22) Date of filing : **07.11.91**

(51) Int. Cl.[5] : **C07D 491/10,** A61K 31/415, // (C07D491/10, 311:00, 235:00)

(30) Priority : **07.11.90 JP 301517/90**

(43) Date of publication of application : **13.05.92 Bulletin 92/20**

(84) Designated Contracting States : **AT BE CH DE DK ES FR GB GR IT LI LU NL SE**

(71) Applicant : **SANWA KAGAKU KENKYUSHO CO., LTD.**
**No. 35, Higashi-sotobori-cho**
**Higashi-ku Nagoya-shi Aichi-ken (JP)**

(72) Inventor : **Kurono, Masayasu, Sanwa Kagaku, Kenkyusho Co.Ltd.**
**Higashi-sotobori-cho 35, Higashi-ku**
**Nagoya-shi, Aichi-ken (JP)**
Inventor : **Miura, Kenji, Sanwa Kagaku, Kenkyusho Co.Ltd.**
**Higashi-sotobori-cho 35, Higashi-ku**
**Nagoya-shi, Aichi-ken (JP)**
Inventor : **Kondo, Yasuaki, Sanwa Kagaku, Kenkyusho Co.Ltd.**
**Higashi-sotobori-cho 35, Higashi-ku**
**Nagoya-shi, Aichi-ken (JP)**
Inventor : **Usui, Toshinao, Sanwa Kagaku, Kenkyusho Co.Ltd.**
**Higashi-sotobori-cho 35, Higashi-ku**
**Nagoya-shi, Aichi-ken (JP)**
Inventor : **Hayashi, Motohide, Sanwa Kagaku, Kenkyusho Co.Ltd.**
**Higashi-sotobori-cho 35, Higashi-ku**
**Nagoya-shi, Aichi-ken (JP)**
Inventor : **Suzuki, Tunemasa, Sanwa Kagaku, Kenkyusho Co.Ltd.**
**Higashi-sotobori-cho 35, Higashi-ku**
**Nagoya-shi, Aichi-ken (JP)**
Inventor : **Tomiya, Noboru, Sanwa Kagaku, Kenkyusho Co.Ltd.**
**Higashi-sotobori-cho 35, Higashi-ku**
**Nagoya-shi, Aichi-ken (JP)**
Inventor : **Nakamura, Shigeyoshi, Sanwa Kagaku, Kenkyusho Co.**
**Higashi-sotobori-cho 35, Higashi-ku**
**Nagoya-shi, Aichi-ken (JP)**
Inventor : **Tanaka, Yukiya, Sanwa Kagaku, Kenkyusho Co.Ltd.**
**Higashi-sotobori-cho 35, Higashi-ku**
**Nagoya-shi, Aichi-ken (JP)**
Inventor : **Sawai, Kiichi, Sanwa Kagaku, Kenkyusho Co.Ltd.**
**Higashi-sotobori-cho 35, Higashi-ku**
**Nagoya-shi, Aichi-ken (JP)**

(74) Representative : **Moore, Anthony John et al**
**Gee & Co. Chancery House Chancery Lane**
**London WC2A 1QU (GB)**

(54) **Hydantoin derivatives, and their use in preventing and treating diabetic complications and circulatory organ diseases.**

(57) Novel hydantoin derivatives have the following general formula (I) :

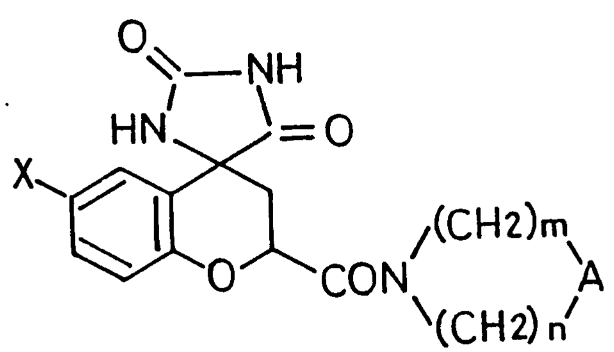

(I)

wherein A represents an oxygen or sulfur atom or the group $B(CH_2)_pY$ in which B is CH or N, Y is a hydrogen atom, an aromatic ring or an alkyl or alkenoyl group, which may or may not be substituted by

EP 0 485 217 A2

an aromatic ring, and p is 0, 1, 2 or 3, X represents a halogen atom, and m and n, which may be identical or different, are each 1, 2 or 3. The components (I) and their salts are efficacious against diabetic complications and circulatory organ diseases.

The present invention relates generally to hydantoin derivatives tne their salts, and more particularly to the use of pharmaceutical compositions containing such compounds as an effective ingredient for preventing and treating diabetic complications and circulatory organ diseases.

Various intensive studies have been made of anti-diabetic medicaments which produce their effects when orally administered. In consequence, pharmaceutical compositions containing sulfornylurea compounds, meso-oxalates and guanidine derivatives have been developed and are now being used as anti-diabetic drugs.

However, they are nothing more than nosotropic medicaments for hyper blood sugar. In particular, diabetes is often accompanied by incurable complications, typically diabetic cataract, neuropathy and retinopathy, but there is little medication for such complications. Nor is there any efficacious and well-established pharmacotherapy.

Some studies of diabetic complications have concentrated on examining inhibitors for aldose reductase which is an enzyme for reducing aldose to, e.g., glucose or galactitol in the human or other animal body. This is because such complications have been known to occur when sorbitol and galactitol produced under the action of this enzyme is built up in the crystalline lens, peripheral nerves, the kidney or other organs - see "Jap. J. Opthalmol.", Vol. 20, page 399 (1976), " Int. Congr. Ser. Excerpta Med.", Vol. 403, page 594 (1977) and "Metabolism", Vol. 28, page 456 (1979 ).

Many diabetics have been reported to have a critical disposition to circulatory organ diseases - see "A Handbook to the Treatment and Control of Diabetic Blood Vessel Complications", 1st Edition (1987), published by Ishiyaku Shuppan, "Br. Med. J.", Vol. 294, page 1443 (1987) and "N. Engl. Med.", Vol. 313, page 1617 (1987). The development of medicaments efficacious against both diabetes and circulatory organ diseases which have a close correlation with diabetes has long been considered desirable, but until now this desideratum has remained unfulfilled.

A primary object of this invention is to provide a novel aldose reductase inhibitor which also has an action on the circulatory organ system, thereby inhibiting the accumulation of sorbitol and galactitol in the body and preventing the occurrence of circulatory organ diseases; both diabetic complications and circulatory organ diseases can thus be prevented from occurring and treated.

According to one aspect of this invention, there is provided a novel hydantoin derivative represented by the following general Formula (I):

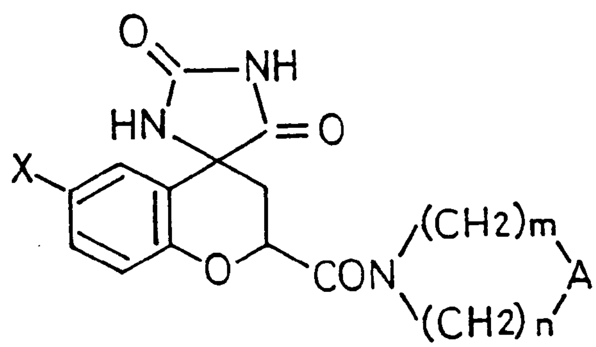

(I)

wherein A represents an oxygen or sulfur atom or the group $B(CH_2)_pY$ in which B is CH or N, Y is a hydrogen atom, an aromatic ring or an alkyl or alkenoyl group, which may or may not be substituted by an aromatic ring, and p is 0, 1, 2 or 3, X represents a halogen atom, and m and n, which may be identical or different, are each 1, 2 or 3; and pharmacologically acceptable salts thereof.

According to another aspect of this invention, there is provided a pharmaceutical composition containing a hydantoing derivative of the general Formula (I) or salt thereof as an effective ingredient, which is effective for the prevention and treatment of diabetic complications and circulatory organ diseases.

The term "alkyl group" as used herein means a straight chain, branched chain or cyclic alkyl group. Preferably, the straight chain alkyl group has 1 to 6 carbon atoms and is, for example, methyl, ethyl, n-propyl, n-butyl, n-pentyl or n-hexyl. Preferably, the branched chain alkyl group again has 1 to 6 cabon atoms, and is, for example, isopropyl, isobutyl, sec.-butyl or tert.-butyl. The cycloalkyl group has at least three carbon atoms and is, for example, cyclopropyl, cyclobutyl, cyclopentyl or cyclohexyl, and may be substituted by an aromatic ring such as a phenyl, pyridyl, thienyl or furyl group which may or may not be substituted by a fluorine, chlorine or bromine atom, or a methyl or methoxy group.

Preferably, the alkenyl group has 2 to 6 carbon atoms and may be, for example, vinyl, allyl, isopropenyl, butenyl, 1,3-butadienyl, isopentenyl or cyclohexenyl, and be substituted in the same way as the cycloalkyl group above.

Preferably, the alkenoyl group has 3 to 5 carbon atoms and may be, for example, 2-propenyl, 2-butenoyl, 3-butenoyl, 2-isobutenoyl or 2-isopentenoyl. This alkenoyl group may again be similarly substituted.

The halogen atom represented by X in Formula (I) may be fluorine, chlorine, bromine and iodine, but particular preference is given to fluorine.

The hydantoin derivatives of Formula (I) have extremely low toxicity when orally administered; function well to inhibit aldose reductase; and exhibit significant effects in, inter alia, inhibiting platelet aggregation, vaso-dilation, depressing blood pressure, and increasing blood-stream. Thus, the compounds of this invention are efficacious for preventing and treating diabetic complications, and may be used for treating, alleviating and preventing various forms of thrombosis, hypertension, cerebrovascular disease and heart disease, typically cerebral infarction, cerebral thrombosis, transient cerebral ischemia, cardiac infarction, coronary thrombosis, arterial sclerosis and stenocardia. For details information on the pharmaceutical and pharmacological actions of these compounds, see the experimental Examples to be given later.

Preferred compounds of this invention are listed below:

1) 4-benzyl-1-{(2S, 4S)-6-fluoro-2′,5′-dioxospiro[chromane-4,4′-imidazolidine]-2-carbonyl}piperidine
2) 4-benzyl-1-{(2R, 4R)-6-fluoro-2′,5′-dioxospiro[chromane-4,4′-imidazolidine]-2-carbonyl}piperidine
3) 4-benzyl-1-{(2RS, 4RS)-6-fluoro-2′,5′-dioxospiro[chromane-4,4′-imidazolidine]-2-carbonyl}piperidine
4) 4-benzyl-1-{(2RS, 4SR)-6-fluoro-2′,5′-dioxospiro[chromane-4,4′-imidazolidine]-2-carbonyl}piperidine
5) 4-(p-fluorobenzyl)-1-{(2S, 4S)-6-fluoro-2′,5′-dioxospiro [chromane-4,4′-imidazolidine]-2-carbonyl}piperidine
6) 4-(p-fluorobenzyl)-1-{(2R, 4R)-6-fluoro-2′,5′-dioxospiro [chromane-4,4′-imidazolidine]-2-carbonyl}piperidine
7) 4-(p-fluorobenzyl)-1-{(2RS, 4RS)-6-fluoro-2′,5′-dioxospiro[chromane-4,4′-imidazolidine]-2-carbonyl}piperidine
8) 4-(p-fluorobenzyl)-1-{(2RS, 4SR)-6-fluoro-2′,5′-dioxospiro[chromane-4,4′-imidazolidine]-2-carbonyl}piperidine
9) 4-(p-chlorobenzyl)-1-{(2S, 4S)-6-fluoro-2′,5′-dioxospiro [chromane-4,4′-imidazolidine]-2-carbonyl}piperidine
10) 4-(p-chlorobenzyl)-1-{(2R, 4R)-6-fluoro-2′,5′-dioxospiro [chromane-4,4′-imidazolidine]-2-carbonyl}piperidine
11) 4-(p-chlorobenzyl)-1-{(2RS, 4RS)-6-fluoro-2′,5′-dioxospiro[chromane-4,4′-imidazolidine]-2-carbonyl} piperidine
12) 4-(p-chlorobenzyl)-1-{(2RS, 4SR)-6-fluoro-2′,5′-dioxospiro[chromane-4,4′-imidazolidine]-2-carbonyl} piperidine
13) 4-(p-methoxybenzyl)-1-((2S, 4S)-6-fluoro-2′,5′-dioxospiro[chromane-4,4′-imidazolidine]-2-carbonyl} piperidine
14) 4-(p-methoxybenzyl)-1-{(2R, 4R)-6-fluoro-2′,5′-dioxospiro[chromane-4,4′-imidazolidine]-2-carbonyl} piperidine
15) 4-(p-methoxybenzyl)-1-{(2RS, 4RS)-6-fluoro-2′,5′-dioxospiro[chromane-4,4′-imidazolidine]-2-carbonyl} piperidine
16) 4-(p-methoxybenzyl)-1-{(2RS, 4SR)-6-fluoro-2′,5′-dioxospiro[chromane-4,4′-imidazolidine]-2-carbonyl} piperidine
17) 4-diphenylmethyl-1-{(2S, 4S)-6-fluoro-2′,5′-dioxospiro[chromane-4,4′-imidazolidine]-2-carbonyl}piperazine
18) 4-diphenylmethyl-1-{(2R, 4R)-6-fluoro-2′,5′-dioxospiro[chromane-4,4′-imidazolidine]-2-carbonyl}piperazine
19) 4-diphenylmethyl-1-{(2RS, 4RS)-6-fluoro-2′,5′-dioxospiro[chromane-4,4′-imidazolidine]-2-carbonyl}piperazine
20) 4-diphenylmethyl-1-{(2RS, 4SR)-6-fluoro-2′,5′-dioxospiro[chromane-4,4′-imidazolidine]-2-carbonyl}piperazine
21) 4-{bis(p-fluorophenyl)methyl}-1-{(2S, 4S)-6-fluoro-2′,5′dioxospiro[chromane-4,4′-imidazolidine]-2-carbonyl} piperazine
22) 4-{bis(p-fluorophenyl)methyl}-1-{(2R, 4R)-6-fluoro-2′,5′-dioxospiro[chromane-4,4′-imidazolidine]-2-carbonyl} piperazine
23) 4-{bis(p-fluorophenyl)methyl}-1-{(2RS, 4RS)-6-fluoro-2′,5′-dioxospiro[chromane-4,4′-imidazolidine]-2-carbonyl}piperazine
24) 4-{bis(p-fluorophenyl)methyl}-1-{(2RS, 4SR)-6-fluoro-2′,5′-dioxospiro[chromane-4,4′-imidazolidine]-2-carbonyl} piperazine
25) 4-phenethyl-1-{(2S, 4S)-6-fluoro-2′,5′-dioxospiro [chromane-4,4′-imidazolidine]-2-carbonyl}piperidine

26) 4-phenethyl-1-{(2R, 4R)-6-fluoro-2′,5′-dioxospiro [chromane-4,4′-imidazolidine]-2-carbonyl}piperidine
27) 4-phenethyl-1-{(2RS, 4RS)-6-fluoro-2′,5′-dioxospiro [chromane-4,4′-imidazolidine]-2-carbonyl}piperidine
28) 4-phenethyl-1-{(2RS, 4SR)-6-fluoro-2′,5′-dioxospiro [chromane-4,4′-imidazolidine]-2-carbonyl}piperidine
29) 4-diphenylmethyl-1-{(2S, 4S)-6-fluoro-2′,5′-dioxospiro[chromane-4,4′-imidazolidine]-2-carbonyl}piperidine
30) 4-diphenylmethyl-1-{(2R, 4R)-6-fluoro-2′,5′-dioxospiro[chromane-4,4′-imidazolidine]-2-carbonyl}piperidine
31) 4-diphenylmethyl-1-{(2RS, 4RS)-6-fluoro-2′,5′-dioxospiro[chromane-4,4′-imidazolidine]-2-carbonyl}piperidine
32) 4-diphenylmethyl-1-{(2RS, 4SR)-6-fluoro-2′,5′-dioxospiro[chromane-4,4′-imidazolidine]-2-carbonyl}piperidine
33) 4-{bis(p-fluorophenyl)methyl}-1-{(2S, 4S)-6-fluoro-2′,5′dioxospiro[chromane-4,4′-imidazolidine]-2-carbonyl} piperidine
34) 4-{bis(p-fluorophenyl)methyl}-1-{(2R, 4R)-6-fluoro-2′,5′dioxospiro[chromane-4,4′-imidazolidine]-2-carbonyl} piperidine
35) 4-{bis(p-fluorophenyl)methyl}-1-{(2RS, 4RS)-6-fluoro2′,5′-dioxospiro[chromane-4,4′-imidazolidine]-2-carbonyl}piperidine
36) 4-{bis(p-fluorophenyl)methyl}-1-{(2RS, 4SR)-6-fluoro-2′,5′-dioxospiro[chromane-4,4′-imidazolidine]-2-carbonyl}piperidine
37) 4-diphenylethyl-1-{(2S, 4S)-6-fluoro-2′,5′-dioxospiro[chromane-4,4′-imidazolidine]-2-carbonyl}piperidine
38) 4-diphenylethyl-1-{(2R, 4R)-6-fluoro-2′,5′-dioxospiro[chromane-4,4′-imidazolidine]-2-carbonyl}piperidine
39) 4-diphenylethyl-1-{(2RS, 4RS)-6-fluoro-2′,5′-dioxospiro[chromane-4,4′-imidazolidine]-2-carbonyl}piperidine
40) 4-diphenylethyl-1-{(2RS, 4SR)-6-fluoro-2′,5′-dioxospiro[chromane-4,4′-imidazolidine]-2-carbonyl}piperidine
41) 4-benzyl-1-{(2S, 4S)-6-fluoro-2′,5′-dioxospiro[chromane-4,4′-imidazolidine]-2-carbonyl}piperazine
42) 4-benzyl-1-{(2R, 4R)-6-fluoro-2′,5′-dioxospiro[chromane-4,4′-imidazolidine]-2-carbonyl}piperazine
43) 4-benzyl-1-{(2RS, 4RS)-6-fluoro-2′,5′-dioxospiro [chromane-4,4′-imidazolidine]-2-carbonyl}piperazine
44) 4-benzyl-1-{(2RS, 4SR)-6-fluoro-2′,5′-dioxospiro [chromane-4,4′-imidazolidine]-2-carbonyl}piperazine
45) 4-(p-fluorobenzyl)-1-{(2S, 4S)-6-fluoro-2′,5′-dioxospiro [chromane-4,4′-imidazolidine]-2-carbonyl}piperazine
46) 4-(p-fluorobenzyl)-1-{(2R, 4R)-6-fluoro-2′,5′-dioxospiro [chromane-4,4′-imidazolidine]-2-carbonyl}piperazine
47) 4-(p-fluorobenzyl)-1-{(2RS, 4RS)-6-fluoro-2′,5′-dioxospiro[chromane-4,4′-imidazolidine]-2-carbonyl} piperazine
48) 4-(p-fluorobenzyl)-1-{(2RS, 4SR)-6-fluoro-2′,5′-dioxospiro[chromane-4,4′-imidazolidine]-2-carbonyl} piperazine
49) 4-phenethyl-1-{(2S, 4S)-6-fluoro-2′,5′-dioxospiro [chromane-4,4′-imidazolidine]-2-carbonyl}piperazine
50) 4-phenethyl-1-{(2R, 4R)-6-fluoro-2′,5′-dioxospiro [chromane-4,4′-imidazolidine]-2-carbonyl}piperazine
51) 4-phenethyl-1-{(2RS, 4RS)-6-fluoro-2′,5′-dioxospiro [chromane-4,4′-imidazolidine]-2-carbonyl}piperazine
52) 4-phenethyl-1-{(2RS, 4SR)-6-fluoro-2′,5′-dioxospiro [chromane-4,4′-imidazolidine]-2-carbonyl}piperazine
53) 4-diphenylethyl-1-{(2S, 4S)-6-fluoro-2′,5′-dioxospiro[chromane-4,4′-imidazolidine]-2-carbonyl}piperazine
54) 4-diphenylethyl-1-{(2R, 4R)-6-fluoro-2′,5′-dioxospiro[chromane-4,4′-imidazolidine]-2-carbonyl}piperazine
55) 4-diphenylethyl-1-{(2RS, 4RS)-6-fluoro-2′,5′-dioxospiro[chromane-4,4′-imidazolidine]-2-carbonyl}piperazine
56) 4-diphenylethyl-1-{(2RS, 4SR)-6-fluoro-2′,5′-dioxospiro[chromane-4,4′-imidazolidine]-2-carbonyl}piperazine
57) 4-cinnamyl-1-{(2R, 4R)-6-fluoro-2′,5′-dioxospiro [chromane-4,4′-imidazolidine]-2-carbonyl}piperazine
58) 4-cinnamyl-1-{(2R, 4R)-6-fluoro-2′,5′-dioxospiro [chromane-4,4′-imidazolidine]-2-carbonyl}piperazine
59) 4-cinnamyl-1-{(2RS, 4RS)-6-fluoro-2′,5′-dioxospiro [chromane-4,4′-imidazolidine]-2-carbonyl}piperazine

60) 4-cinnamyl-1-{(2RS, 4SR}-6-fluoro-2′,5′-dioxospiro [chromane-4,4′-imidazolidine]-2-carbonyl}piperazine

61) 4-(3,4,5-trimethoxycinnamoyl)-1-{(2S, 4S)-6-fluoro-2′,5′-dioxospiro[chromane-4,4′-imidazolidine]-2-carbonyl} piperazine

62) 4-(3,4,5-trimethoxycinnamoyl)-1-{(2R, 4R)-6-fluoro-2′,5′-dioxospiro[chromane-4,4′-imidazolidine]-2-carbonyl} piperazine

63) 4-(3,4,5-trimethoxycinnamoyl)-1-{(2SR, 4SR)-6-fluoro-2′,5′-dioxospiro[chromane-4,4′-imidazolidine]-2-carbonyl}piperazine

64) 4-(3,4,5-trimethoxycinnamoyl)-1-{(2SR, 4RS)-6-fluoro-2′,5′-dioxospiro[chromane-4,4′-imidazolidine]-2-carbonyl}piperazine

65) 4-cinnamoyl-1-{(2S, 4S)-6-fluoro-2′,5′-dioxospiro [chromane-4,4′-imidazolidine]-2-carbonyl}piperazine

66) 4-cinnamoyl-1-{(2R, 4R}-6-fluoro-2′,5′-dioxospiro [chromane-4,4′-imidazolidine]-2-carbonyl}piperazine

67) 4-cinnamoyl-1-{(2SR, 4SR)-6-fluoro-2′,5′-dioxospiro [chromane-4,4′-imidazolidine]-2-carbonyl}piperazine

68) 4-cinnamoyl-1-{(2SR, 4RS)-6-fluoro-2′,5′-dioxospiro [chromane-4,4′-imidazolidine]-2-carbonyl}piperazine

69) 4-(4-methoxycinnamoyl)-1-{(2S, 4S)-6-fluoro-2′,5′-dioxospiro[chromane-4,4′-imidazolidine]-2-carbonyl} piperazine

70) 4-(4-methoxycinnamoyl)-1-{(2R, 4R)-6-fluoro-2′,5′-dioxospiro[chromane-4,4′-imidazolidine]-2-carbonyl} piperazine

71) 4-(4-methoxycinnamoyl)-1-{(2SR, 4SR)-6-fluoro-2′,5′-dioxospiro[chromane-4,4′-imidazolidine]-2-carbonyl} piperazine

72) 4-(4-methoxycinnamoyl)-1-{(2SR, 4RS)-6-fluoro-2′,5′-dioxospiro[chromane-4,4′-imidazolidine]-2-carbonyl} piperazine

73) 4-(4-fluorocinnamoyl)-1-{(2S, 4S)-6-fluoro-2′,5′-dioxospiro[chromane-4,4′-imidazolidine]-2-carbonyl} piperazine

74) 4-(4-fluorocinnamoyl)-1-{(2R, 4R)-6-fluoro-2′,5′-dioxospiro[chromane-4,4′-imidazolidine]-2-carbonyl} piperazine

75) 4-(4-fluorocinnamoyl)-1-{(2SR, 4SR)-6-fluoro-2′,5′-dioxospiro[chromane-4,4′-imidazolidine]-2-carbonyl} piperazine

76) 4-(4-fluorocinnamoyl)-1-{(2SR, 4RS)-6-fluoro-2′,5′-dioxospiro[chromane-4,4′-imidazolidine]-2-carbonyl} piperazine

The compounds represented by Formula I according to this invention may exist in the form of a solvate, e.g. a hydrate or salt.

In view of their application, the salts of the compounds according to this invention should preferably be in pharmaceutically acceptable form. For example, in the form of a salt of an inorganic acid such as phosphoric acid, hydrochloric acid, sulfuric acid, hydrogen bromide or hydrogen iodide or an organic acid such as malonic, acetic, succinic, maleic, fumaric, lactic, tartaric, citric or methanesulfonic acid. In addition, the compounds of the Formula (I) may be in the form of a pharmaceutically acceptable salt of an inorganic or organic base such as their sodium, lithium, potassium or quaternary ammonium salt or their diethanolamine salt. These salts may be easily obtained by treating the compounds of Formula (I) with a suitable acid or base.

The compounds of Formula (I) have two asymmetric carbon atoms per structure and so can give two stereoisomers and their optical isomers, which are all included in the scope of this invention.

The compounds according to this invention may be synthesized by any one appropriate procedure, for example procedures analogous to those disclosed in JP-P-61(1986)-200991, JP-A-63(1988)-57588 and JP-A-1(1989)-242301. Set out below are some preferred examples of synthesis.

The compounds having Formula (I) may be obtained by the reaction of a carboxylic acid represented by the following general Formula (II):

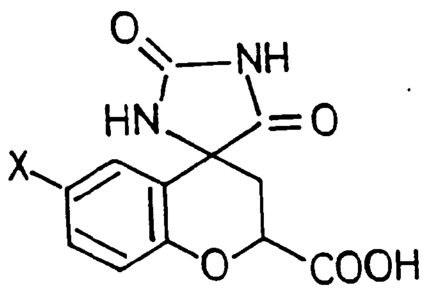

(II)

wherein X has the previous meaning with an amino group-containing compound having the following general Formula (III):

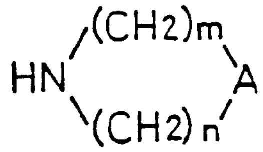

(III)

wherein A, n and m have the previous meanings under conditions suitable for a carboxyl-imino condensation reaction.

The above passage, "the reaction ... under conditions suitable for a carboxyl-imino condensation reaction", is to be understood to include not only the reactions in the presence of catalysts between the carboxylic acid (II) and the compound (III) in which they are left intact, but also reactions between both the compounds, at least one of which is converted into its functional derivative, and any other suitable reaction scheme.

The functional derivatives of the carboxylic acids (II) may be salts, acid halides, active esters, acid anhydrides, etc., and those obtained with carbomyl chloride, aminoborane, etc. for the compounds (III).

Typically, the compounds (I) may be obtained by the direct reaction between the carboxylic acid (II) and the compounds (III) in the presence of a catalyst, such as $TiCl_4$ or $SiCl_4$DCC. This reaction proceeds easily and smoothly at a temperature of -20 to 150°C, preferably 0 to 50°C, in a solvent which takes no part in it, e.g. a halogen solvent such as dichloromethane or chloroform, a basic solvent such as pyridine or an aprotic solvent such as DMSO or DMF.

Alternatively, the compounds (I) may be obtained by the reaction of a compound (III) with a compound having the following general formula (IV):

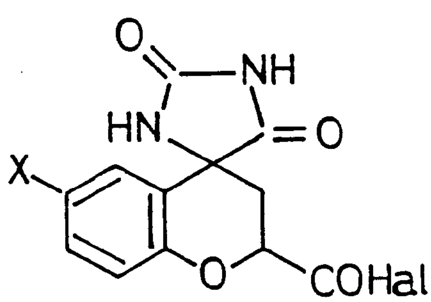

(IV)

wherein X has the previous meaning and Hal is a halogen atom, e.g. F, Cl, Br or I. This reaction proceeds easily and smoothly at a temperature of -20 to 150°C, preferably 0 to 30°C in a solvent, e.g. a halogen solvent such as dichloromethane or chloroform, a basic solvent such as pyridine or an aprotic solvent such as DMSO or DMF.

The compounds (IV) may be obtained by converting a carboxylic acid (II) into the corresponding acid halide with a suitable halogenating agent such as thionyl chloride, thionyl bromide, phosphorus trichloride, phosphorus pentachloride or phosphorus tribromide.

Furthermore, the compound (I) may be obtained by the reaction of a compound (III) with a compound having the following general Formula (V):

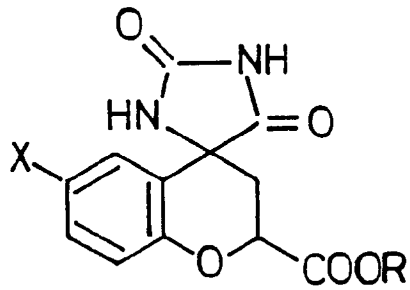

(V)

wherein X has the previous meaning and R represents a hydrocarbon group having 1 to 5 carbon atoms, e.g. methyl, ethyl, propyl, butyl or pentyl. This reaction proceeds easily and smoothly at a temperature of 0 to 150°C, preferably 20 to 100°C, in a solvent, e.g. an alcohol such as methanol, ethanol or propanol, a halogen solvent such as dichloromethane or chloroform, a basic solvent such as pyridine or an aprotic solvent such as DMSO or DMF.

Pharmaceutical compositions containing as an effective ingredient a compound of Formula (I) or its pharmaceutically and pharmacologically acceptable salt or solvate may be formulated for oral, buccal, topical or per rectal administration. In other words the compositions may be formulated by conventional procedures in the form of either solid preparations such as tablets, pills, capsules, powders, granules or suppositories, or liquid preparations such as folutions, suspensions or emulsions. The solid preparations may be obtained using such vehicles or excipients as starch, lactose, glucose, calcium phosphate, magnesium stearate or gum arabic, optionally with lubricants, binders, disintegrators, colorants or the like. The liquid preparations may be obtained using stabilizers, dissolution aids, suspension agents, emulsifiers, buffers, preservatives and the like.

The compounds of this invention or their salts may be administered to the subject by either oral or parenteral (e.g. intramuscular, hypodermic and intravenous) routes. The dose may be determined case-by-case depending upon what type and form of preparation are used, what conditions patients are in, how old they are and other significant factors. Usually, human adults may receive a dose of about 0.1 to 2000 mg, particularly about 1 to 500 mg a day and one to three times on a daily basis.

EXAMPLES

The present invention will now be illustrated with reference to the following Examples.

Preparative Example 1 - Synthesis of Starting Material

Preparation of (2S,4S)-6-fluoro-2′,5′-dioxospiro[chromane-4,4′-imidazolidine]-2-carbonyl chloride

Four hundred (400) ml (5.11 mol) of thionyl chloride were added to 20.0 g (67.1 mmol) of (2S,4S)-6-fluoro-2′,5′-dioxospiro[chromane-4,4′-imidazolidine]-2-carboxylic acid, and the solution was stirred under reflux for 18 hours. After that, the solution was dried to solid under reduced pressure to obtain the captioned 2-carbonyl chloride product in an amount of 20.0 g (in a 100 % yield).

In order to prepare the following compound, this solid compound was used as such or without any additional purification.

Compound 1

Preparation of 4-benzyl-1-{(2S,4S)-6-fluoro-2′,5′-dioxospiro[chromane-4,4′-imidazolidine]-2-carbonyl} piperazine

Ten (10.0) g (57.1 mmol) of 4-benzylpiperidine, 11.5 g (114 mmol) of triethylamine and 100 ml (1.29 mol) of N,N-dimethylformamide were cooled down to - 30°C, and added to this over 30 minutes was a solution of 17.0 g (57.0 mmol) of the 2-carbonyl chloride synthesized in Preparative Ex. 1 in 100 ml (1.29 mol) of N,N-dimethylformamide. The solution was stirred at 15 to 25°L for a further 15 hours, after which the solvent was removed by distillation. The resulting product was purified by silica gel chromatography ($CH_2Cl_2$/MeOH = 9/1) and dried to solid under reduced pressure to obtain 20.4 g of the captioned compound (in an 82.0 % yield) in the form of a colorless amorphous material).

Melting Point: 144-6°C

IR spectra ( $\nu^{KBr}_{max}$ ) $cm^{-1}$
3220, 1780, 1735, 1640, 1495
NMR spectra (DMSO-$d_6$)δ ppm
1.21 - 3.57 (12H, m)
4.03 - 4,08 (1H, m)
5.67 - 5.72 (1H, m)
7.10 - 7.50 (8H, m)
8.58 (1H, s)
11.21 (1H, s)
Mass spectra
(EI/DI)m/z : 437 ($M^+$)
202 (base peak)

Compound 2

Preparation of 4-diphenylmethyl-1-{(2S,4S)-6-fluoro-2,5′-dioxospiro[chromane-4,4′-imidazolidine]-2-carbonyl}piperazine

Seventeen (17.0) g (67.5 mmol) of N-diphenylmethylpiperazine, 12.3 g (80.9 mmol) of 1,8-diazabicyclo[5,4,0]-7-undecene and 90.0 ml (1.16 mol) of N,N-dimethylformamide were cooled down to - 30°C, and added to them over 30 minutes was a solution of 20.0 g (67.0 mmol) of the 2-carbonyl chloride product synthesized in Preparative Example 1 in 90.0 ml (1.16 mol) of N,N-dimethylformamide. After the solution was stirred at 15 to 25°C for a further 15 hours, the solvent was removed by distillation. The resulting product was purified by silica gel chromatography ($CH_2Cl_2$/MeOH = 9/1) and dried into solid under reduced pressure to obtain 28.0 g of the captioned compound (in an 80.7 % yield) in the form of a colorless amorphous material.
Melting Point: up to 180°C
IR spectra ( $\nu^{KBr}_{max}$ ) $cm^{-1}$
3400, 3060, 1780, 1735, 1650, 1400
NMR spectra (DMSO-$d_6$)δ ppm
2.14 - 3.91 (10H, m)
4.52 (1H, s)
5.62 - 5.67 (1H, m)
7.06 - 7.62 (13H, m)
8.53 (1H, s)
11.15 (1H, s)
Mass spectra
(EI/DI)m/z : 514 ($M^+$)
167 (base peak)

Compound 3

Preparation of 4-cinnamyl-1-{(2S,4S)-6-fluoro-2′,5′-dioxospiro[chromane-4,4′-imidazolidine]-2-carbonyl}piperazine

The procedures of Preparative Example 1 were followed with 4-cinnamylpiperazine and the 2-carbonyl chloride product synthesized in Preparative Ex.1, thereby obtaining the captioned compound.
Yield : 77.3 %
Melting Point: 228-33°C
IR spectra ( $\nu^{KBr}_{max}$ ) $cm^{-1}$
3181, 1775, 1730, 1640, 1484
NMR spectra (DMSO-$d_6$)δ ppm
2.33 - 3.73 (12H, m)
5.66 - 5.71 (1H, m)
6.40 - 6.50 (1H, m)
6.68 - 6.74 (1H, m)
7.08 - 7.60 (8H, m)
8.49 (1H, s)

11.12 (1H, bs)
Mass spectra
(EI/DI)m/z : 464 ($M^+$)
127 (base peak)

Test Example 1 : Action on Aldose Reductase Inhibition

Compounds 1-3 were tested, using sorbinil as a control compound, according to the method described by P. F. Kador et al "Biophys. Chem.", Vol. 8, page 81 (1978) to detrermine how much they inhibited aldose reductase.

Table 1 sets out the results, from which it is noted that the present compounds can inhibit aldose reductase much more significantly than ever before

Table 1

| Compounds | $IC_{50}$ (M) |
|---|---|
| Control - Sorbinil | $2.0 \times 10^{-7}$ |
| Compound 1 | $3.1 \times 10^{-7}$ |
| 2 | $4.5 \times 10^{-8}$ |
| 3 | $4.1 \times 10^{-7}$ |

Note that $IC_{50}$ is an index to the 50 % inhibition of aldose reductase.

Test Example 2 : In-Vitro Action on Inhibiting Platelet Aggregation

Added to blood gathered from the descending aorta of a lightly etherized rat was citric acid (3.8 % sodium citrate) in a volume ratio of 1/10, and the blood was centrifuged at 4°C and 1500 rpm for 10 minutes to obtain a supernatant fluid in the form of platelet rich plasma (PRP for short). Then, the residues were centrifuged to obtain a supernatant fluid in the form of platelet poor plasma (PPP for short). For measurement, an amount of PRP collected from two to four animals was pooled. The platelet aggregation test was carried out with NBS HEMA TRACER (Model PAT-60 made by Niko Bioscience Co., Ltd.). 267 µml of PRP were put in a PRP cuvette having a stirrer bar with the transmittance set at 0%, while 300 µl of PPP were put in a PPP cuvette with the transmittance set at 100%. After 1-minute preincubation, 3 µl of the test compound were added and, one minute later, 30 µl of an aggregation inducer (collagen or ADP) were introduced. Added to a control group was the test compound together with 3 µl of dimethyl sulfoxide. Upon the addition of the aggregation inducer, a maximum transmittance change was read to find a platelet aggregation change in %. An inhibition in % relative to the control - 100% was found to calculate the 50% inhibition concentration ($IC_{50}$). Table 2 sets out the results, from it is noted that the present compounds have a significant action on inhibiting platelet aggregation.

Table 2

| Compounds | $IC_{50}$ (M) | |
|---|---|---|
| | Collagen | ADP |
| Indomethacin | $6.8 \times 10^{-5}$ | |
| Aspirin | $2.1 \times 10^{-4}$ | |
| Compound 1 | $6.3 \times 10^{-4}$ | |
| 2 | $4.3 \times 10^{-4}$ | $7.7 \times 10^{-4}$ |
| 3 | $3.7 \times 10^{-4}$ | |

Test Example 3 : Ex-Vivo Actions on Inhibiting Platelet Aggregation as well as Poikilocytes and Erythrocyte Oxygen Dissociation

1) Administration and Blood Gathering

Five minutes after the test compound had been intravenously administered to the tail of a rat, blood was gathered from the descending aorta of the rat which was lightly etherized. Added to this blood was citric acid (3.8% sodium citrate) in a volume ratio of 1/10.

2) Determination of Platelet Aggregation

The thus treated blood was centrifuged at 1500 rpm for 5 minutes to obtain a supernatant fluid in the form of PRP. The residues were then again centrifuged at 4°C and 3000 rpm for 10 minutes to obtain a supernatant fluid in the form of PPP. For the platelet aggregation test, NAB HEMA TRACER 601 (Model PAT-60 made by Niko Bioscience Co., Ltd.) was used. 180 µml of PRP were put in a PRP cuvette having a stirrer bar with the transmittance set at 0%, while 300 µl of PPP were put in a PPP cuvette with the transmittance set at 100%. After 1-minute preincubation, 20 µl of an aggregation inducer (collagen or ADP) were introduced.

A control group, to which dimethylacetamide and tartaric acid in a ratio of 2/3 were given in a quantity of 1 ml/kg, was treated as with the test group. Upon the addition of the aggregation inducer, a maximum transmittance change was read to find a platelet aggregation change in %. An inhibition in % relative to the control - 100% was found to calculate the 50% inhibition concentration ($IC_{50}$). Table 3 sets out the results, from it is noted that the present compounds have a significant action on inhibiting platelet aggregation.

Table 3 - Ex-Vivo Action on Platelet Aggregation

| Compounds | Conc. (mg/kg) | ADP | Collagen |
|---|---|---|---|
| | | ($1 \times 10^{-6}$M) | (10 µM) |
| Compound 1 | 1.0 | 44* | 57 |
| Aspirin | 30.0 | - | 77 |
| Indomethacin | 30.0 | - | 49 |

*;$p<0.05$

3) Determination of Poikilocytes

Blood gathered with the addition of citric acid was centrifuged at 4°C and 1500 rpm for 10 minutes. The

supernatant fluid was discarded. Added to the residues was physiological saline in an amount of about 1.5 times as large, again followed by 10-minute centrifugation at 4°C and 1500 rpm. The supernatant fluid was discarded. One hundred (100) µl of erythrocytes left upon such procedures repeated twice were diluted with physiological saline to prepare 50 ml of a 0.2 % erythrocyte suspension. This suspension was incubated at 37°C for 5 minutes, cooled down to room temperature and examined to find how much the poikilocytes were ameliorated by the filtration-under-pressure procedures described by F. Katsuraya in "Geriatric Medicine", Vol. 20, pp. 151-156 (1982).

To this end, the suspension sample was passed from a continuous feeder through a Millipore membrane having a mean pore size of 5 µl at a rate of 5 ml/min, during which the pressure on the upstream side of the membrane was measured through a pressure transducer with a pressure amplifier and, at the same time, was recorded on a polygraph chart sheet.

The results, set out in Table 4, indicate that the present compounds have a significant action upon ameliorating poikilocytes.

Table 4 - *Ex-Vivo* Action on Ameliorating Poikilocytes

| Compounds | Conc. in mg/kg | Amelioration in % |
|---|---|---|
| Compound 1 | 0.1 | 29.3 |
| 2 | 1.0 | 37.9 |

4) Determination of Oxygen Dissociation

Blood gathered with the addition of heparin was used to determine the oxygen dissociation of the erthrocytes with HEMOX-ANALYZER.

The results, reported below, indicate that the present compound has a remarkable action on the oxygen dissociation of erythrocites.

Table 5 - Action on the Oxygen Dissociation of Erythrocites

| Compounds | Conc. in mg/kg | $P_{50}$ in mmHg | |
|---|---|---|---|
| | | Mean | S.E. |
| Control | - | 31.08 | 0.576 |
| Compound 2 | 10 | 32.58 | 0.170 |

Test Example 4 - Action on Extracted Guinea Pigs' Aortae

Stunned by receiving a blow on the head, Hartley guinea pigs (weighing 300-500 g) were fixed at the back regions, and the thoracic aortae were then extracted to prepare helical samples, each ca. 2 mm in width and ca. 25 mm in length. Each sample was suspended in a Magnus tube under a load of ca. 1 g and connected at the upper end with an FD pickup by means of silk thread to record its isometric tension change on a recorder. Note that the Magnus tube contained 10 ml of a Krebs-Henseleit solution and was held at 37°C with the supply of 95% $O_2$-5% $CO_2$ gases, said Krebs-Henseleit solution consisting of, in mM, the following ingredients: NaCl: 118, KCl: 4.7, $CaCl_2$: 2.55, $MgSO_4$: 1.18, $KH_2PO_4$: 1.18, $NaHCO_3$: 24.88 and glucose: 11.1

Prior to initiating the experiment, the sample was allowed to stand for 60 to 90 minutes, during which the Krebs-Henseleit solution was replaced by a new one at an interval of 20 to 30 minutes. After the tension recorded had been found to be kept stable, KCl was added to obtain a final concentration of 2.5 x $10^{-2}$M or norepinephrine (NE for short) was added to obtain a final concentration of $10^{-6}$ g/ml. After the contraction of the sample had been kept constant, the test compound was added at an incremental rate from the concentration of ca. $10^{-8}$ to $10^{-6}$M to observe what reaction took place. Finally, papaverine was additionally provided to regulate the final concentration to 2 x $10^{-5}$g/ml, thereby observing and recording what relaxing reaction took place.

The state stabilized by the addition of KCl or NE was defined as a 100% relaxation rate, on the basis of

which what reaction took place at each concentration was relatively estimated. For the purpose of data analysis, the 50% relaxation rates ($IC_{50}$) were found.

The results are reported in Table 6.

Table 6

| Compounds | $IC_{50}$ (M) Contraction by KCl | Contraction by NE |
|---|---|---|
| Compound 1 | $1.3 \times 10^{-6}$ | $1 \times 10^{-5}$ |
| 2 | $1.2 \times 10^{-4}$ | $1 \times 10^{-6}$ |
| 3 | $> 10^{-3}$ | $> 10^{-3}$ |
| Cinepazid | $2.0 \times 10^{-3}$ | $1 \times 10^{-3}$ |
| Cinnarizine | $2.0 \times 10^{-4}$ | $1 \times 10^{-4}$ |

Test Example 5 - Action on Extracted Guinea Pigs' Hearts

Stunned by receving a blow on the heads, Hartley guinea pigs (weighing 300-400 g) were fixed on the back regions to extract their hearts. Each of the extracted hearts was placed in a beaker containing the same Krebs-Henseleit solution as used in Test Example 4 and supplied with 95% $O_2$-5% $CO_2$ gases, thereby stripping it of blood deposits, and was then passed into a sample forming glass vessel. This vessel was previously filled with a Krebs-Henseleit solution and the above gaseous mixture continued to be supplied thereto during preparing the sample.

In order to prepare the sample, the right and left atria were cut out of the ventricular muscule along the atriventricular sulcus from the center of both the right and left auricles.

(a) Right Atrium Sample

The thus extracted right atrium was suspended in a Magnus tube with a tension of 0.2 to 0.3 g, said tube supplied with 95% $O_2$ - 5% $CO_2$ gases, containing 10 ml of a Krebs-Henseleit solution and maintained at 32±1°C. The contraction of the sample was recorded on a recorder through an FD pickup simultaneously with counting and recording the beat with an instantaneous cardiograph unit.

(b) Left Atrium Sample

The extracted left atrium was fixed with micro wire clamp through both the auricles in a Magnus tube supplied with 95% $O_2$ - 5% $CO_2$ gases, containing 30 ml of a Krebs-Henseleit solution and maintained at a solution temperature of 32±1°C, and then suspended therein under a checking tension of ca. 0.25 g. While a platinum bipolar electrode attached to the above-mentioned micro wire clamp was in contact with the atrium, stimuli were given to it by 1-Hz, 2-msec. and 10-V rectangular waves derived through an electrical stimulator and an isolator to record the movement of said atrium with an FD pickup connected with it through silk thread.

The actions of the present and control compounds on the right (counting the heart rate) and left (measuring the systole force) atria are reported below.

Table 7

| Compounds | Heart Rates | | Systole Forces | |
|---|---|---|---|---|
| | $10^{-5}$(M) | $10^{-4}$(M) | $10^{-5}$(M) | $10^{-4}$(M) |
| Compound 1 | - | - | - | + |
| 2 | + | ++ | - | + |
| 3 | + | ++ | - | + |
| Control A | ++ | ++ | ++ | ++ |
| B | ++ | ++ | - | + |

Note that A and B stand for propranolol and ifenprodil, respectively, and that ++ indicates 20% or more suppression, + 10-20% suppression and - 0% suppression.

Test Example 6 - Action on the Coronary Bloodstream and Cardiac Function of Dogs under Anesthesia (i.v.)

Fully developed, mongrel male and female dogs were each put under anesthesia with sodium pentobarbital and subjected to commissurotomy while artificial respiration was applied to them. The coronary artery bloodstream was determined with an electromagnetic bloodstream probe attached to the ramus circumflexus of the left coronary artery.

Each of the test compounds was administered to the animal through a cannula fixedly inserted into the cephalic vein.

The results, set out below, indicate that the present compounds serve well to increase the coronary bloodstream.

Table 8

| Compounds | Doses | C.B.S. |
|---|---|---|
| Control | | 8.7±2.1 |
| Compound 1 | 0.1 mg/kg | 9.6±6.9 |
| 2 | 1.0 mg/kg | 13.8±2.9 |

Note that C.B.S. stands for coronary bloodstream.

Pharmaceutical Example 1

The ingredients, shown in Table 9, were blended together and tableted conventionally to prepare 1000 tablets for oral administration. These tablets each contain 50 mg of the active ingredient.

Table 9

| Ingredients | Amounts in g |
|---|---|
| Compound 2 | 50 |
| Sodium citrate | 25 |
| Arginine | 10 |
| Polyvinylpyrrolidone | 10 |
| Magnesium stearate | 5 |

## Claims

1. A hydantoin derivative represented by the following general formula (I):

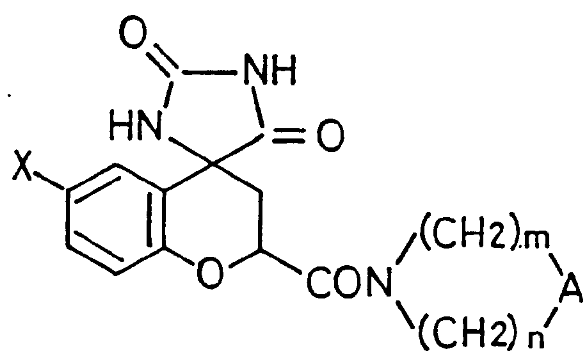

(I)

wherein A represents an oxygen or sulfur atom or the group $B(CH_2)_pY$ in which B is CH or N, Y is a hydrogen atom, an aromatic ring or an alkyl or alkenoyl group, which may or may not be substituted by an aromatic ring, and p is 0, 1, 2 or 3, X represents a halogen atom, and m and n, which may be identical or different, are each 1, 2 or 3; and pharmacologically acceptable salts thereof.

2. A hydantoin derivative as claimed in Claim 1, wherein the alkyl group represented by $R^1$ and $R^2$ is a straight or branched chain alkyl group having from 1 to 6 carbon atoms or a cycloalkyl group having at least three carbon atoms; the alkenyl group has from 2 to 6 carbon atoms; and the alkenoyl group has from 3 to 5 carbon atoms.

3. A hydantoin derivative as claimed in Claim 3, wherein the cycloalkyl, alkanoyl or alkenyl group is substituted with an aromatic ring, which itself may be substituted or unsubstituted.

4. A hydantoin derivative as claimed in Claim 1, being 4-benzyl-1- {(2S,4S)-6-fluoro-2′,5′-dioxospiro[chromane-4,4′-imidazolidine]-2-carbonyl}piperazine; 4-diphenylmethyl-1-{(2S,4S)-6-fluoro-2′,5′-dioxospiro[chromane-4,4′-imidazolidine]-2-carbonyl}piperazine; 4-cinnamyl-1-{(2S,4S)-6-fluoro-2′,5′-dioxospiro[chromane-4,4′-imidazolidine]-2-carbonyl} piperazine; or a pharmaceutically acceptable salt thereof.

5. A composition for the prevention and treatment of diabetic complications and/or circulatory organ diseases, which comprises, as an effective ingredient, at least one hydantoin derivative as claimed in any preceding claim

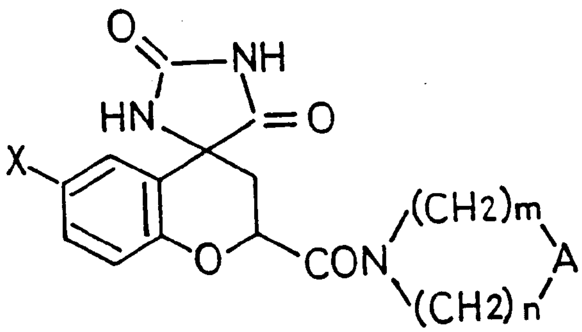

(I)

6. The use, in the manufacture of a medicament for the prevention or treatment of diabetic and/or circulatory organ diseases, of a hydantoin derivative as claimed in any of Claims 1 to 4.